# EUROPEAN PATENT SPECIFICATION

(11) **EP 2 543 045 B1**
(45) Date of publication and mention of the grant of the patent: **07.05.2014**
(21) Application number: 10713117.9
(22) Date of filing: 02.03.2010
(51) Int. Cl.: G21K 1/04

(54) **POSITION DETECTION FOR MULTI-LEAF COLLIMATORS**
POSITIONSDETEKTOR FÜR MEHRBLÄTTRIGE KOLLIMATOREN
DÉTECTION DE POSITION POUR COLLIMATEURS MULTI-LAMES

(43) Date of publication of application: 09.01.2013
(73) Proprietor: Elekta AB (PUBL), 103 93 Stockholm (SE)
(72) Inventor: HUMPHREY, Malcolm, Horsham West Sussex RH12 2NU (GB)
(74) Representative: Downing, Michael Philip
(86) International application number: PCT/EP2010/001265
(87) International publication number: WO 2011/107111

(56) References cited:
- EP-A1- 1 961 446
- EP-A2- 0 314 231
- US-A1- 2004 240 621
- US-A1- 2008 298 553

## Description

### FIELD OF THE INVENTION

The present invention relates to an improved form of position detection, suitable for the detection of leaf positions in a Multi-Leaf Collimator ("MLC") for use in radiotherapeutic apparatus.

### BACKGROUND ART

There are particular difficulties in remotely sensing the positions of individual leaves of a multi leaf collimator (MLC) in a linear accelerator (Linac). An MLC is used to shape the beam of X-ray (or other) radiation produced by a linac, in order to treat a patient. For the control system to deliver a correctly shaped dose, the position of each leaf must be accurately determined. Current methods that are employed use a combination of a standard CCTV camera and reflectors positioned on each leaf, such as in EP0314231, for example. A source of light illuminates the area in which the reflectors lie, and the CCTV camera receives an image that includes the reflections. The position of each leaf can then be determined from the position of each reflection within the CCTV camera image.

The main problem with such systems is that the accuracy and resolution are limited by the number of pixels within the camera. This cannot be easily increased. In addition, most electronics (including CCTV cameras) are susceptible to radiation, and will eventually break down if exposed to significant levels. Radiation hardened cameras can be designed, but these are expensive and are usually bespoke, so are not readily available.

Most CCTV cameras also have a limited frame rate of (typically) 30 frames per second at most. Where fast movements are required of the MLC leaves, this frame rate may not be adequate to keep up.

Video processing software is also required, which is computationally demanding and therefore increases system complexity and cost.

The CCTV camera is bulky, and needs to be fitted into an area where space is at a premium.

Electromagnetic noise generated from within the Linac can interfere with the image quality and may interfere with the image processing software, causing a loss of leaf positions.

Finally CCTV cameras are responsive to a broad range of optical wavelengths, making them susceptible to sources of interference such as room lasers, fluorescent lights, and the like.

### SUMMARY OF THE INVENTION

The present invention therefore provides a multi-leaf collimator comprising an array of laterally spaced elongate leaves each having a longitudinal edge on which is mounted a reflective element, a scanning light source adapted to issue a beam of light in a scanning pattern, so that the beam illuminates a point that moves over the longitudinal edges of a plurality of leaves of the array, and a detector for light reflected from the reflective elements.

The scanning light source can comprise a source of light that illuminates a mirror, the mirror being controllably adjustable so as to direct a reflected beam of light in a scan pattern. The mirror is preferably part of a micro-electromechanical ("MEMS") device, so as to allow swift and accurate control of the mirror position.

At least one other mirror is preferably located (or locatable) in the radiation beam path, to permit the scanning light source to be located out of the beam path thereby protecting it from incident radiation.

The scanning pattern preferably illuminates a point that moves along the longitudinal edges of a plurality of leaves of the array in succession. Other scanning patterns are possible, however.

The scanning light source can comprise a laser.

The reflective element is preferably retro-reflective.

The invention also relates to a radiotherapeutic apparatus comprising such a multi-leaf collimator.

Tracking objects using MEMs mirrors and laser light is known, for example from US 7,498,811 or from "Fast and high-precision 3D tracking and position measurement with MEMs micromirrors" Milanovic, V. & Wing, Kin Lo, IEEE/LEOS International Conference on Optical MEMs and Nanophotonics, 2008 (pp 72-73). However, it does not appear to have been applied to the detection of leaf positions in an MLC array. The Milanovic system also needs to interrogate the MEMS driver in order to ascertain the position of the mirror at the instant when the signal is detected or reflected. By placing reflective elements on the moving object, the complexity and the cost of the system is reduced, and multiple object positions (such as MLC leaves) can be tracked within the laser scanning field of view. In addition, placing so-called "boundary markers" allows the system to determine the object position relative to the boundary markers using simple algorithms based on the signal time measurements, and avoids the need to interrogate the MEMS driver.

### BRIEF DESCRIPTION OF THE DRAWINGS

An embodiment of the present invention will now be described by way of example, with reference to the accompanying figures in which;
Figure 1 shows a known solution based on a video camera;
Figure 2 shows an arrangement according to the present invention;
Figure 3 shows the scanning pattern of figure 2; and
Figure 4 shows an alternative scan pattern.

### DETAILED DESCRIPTION OF THE EMBODIMENTS

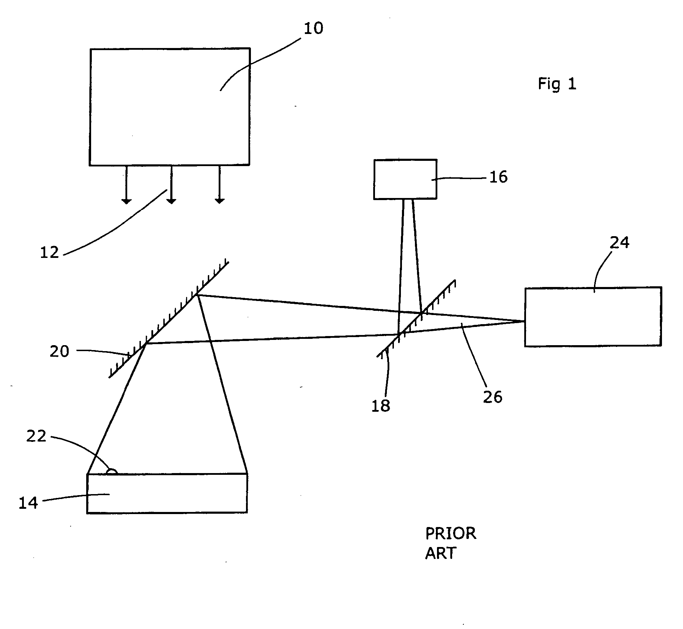

Figure 1 shows a known position tracking system for an array of MLC leaves. A radiation source 10 emits a beam of radiation 12 towards a treatment volume, illustrated (by convention) as being in a downward direction. In practice, the source is usually rotated around the treatment volume so as to irradiate the region of interest from a range of different angles, thereby reducing the irradiation of surrounding tissue.

The shape of the radiation beam 12 is collimated by an MLC comprising an array of individual leaves 14. These are deep in the beam direction, elongate, and narrow in width. They are arranged side-by-side, and are individually moveable back and forth, into and out of the beam. Thus, a large number of such leaves can shape the beam as required. Typical MLCs include 40, 80 or 160 leaves depending on the desired resolution.

The leaves are driven by an electric motor. In order to place the motor out of the beam and protect it from radiation damage, and in order to fit the required number of motors within the available space, the motors are usually spaced some distance from the leaf that they control and the necessary torque is transmitted from the motor to the leaf by a mechanical arrangement. The correct movement of the leaf is therefore subject to correct programming of the motor control unit, correct operation of the motor, and correct operation of whatever mechanical link is employed. It is therefore necessary to verify that the leaf is in fact in the correct position, to allow for failsafe operation of the apparatus.

As shown in figure 1, this is achieved by providing a light source 16 to one side of the radiation source 10, which illuminates the MLC leaves via a pair of periscopically arranged mylar mirrors 18, 20. A reflector 22 is attached to a known location on an upper (in the orientation shown in figure 1) surface of the leaf 14, and reflects light back via the mirrors to a video camera co-located with the light source 16. The camera therefore records an image from which the position of each leaf is obtainable. This can be compared to the intended position of each leaf, and the treatment halted if an error greater than a certain magnitude is detected.

Figure 1 also shows the field lamp 24. This emits a light beam 26 which is incident on the rear of the first mylar mirror 18 through which at least part of the beam is transmitted. That then falls on the second mirror 20 and is reflected toward the treatment volume. Both the source/camera 16 and the field lamp 24 are positioned relative to the radiation source and the mirrors 18, 20 so that they are in a location that is optically identical to that of the radiation source 10. Thus, the camera receives a beam's-eye view of the leaves 14, and the field lamp illuminates the treatment volume in the same manner as the radiation, allowing it to be used for pre-treatment alignment of the patient.

This form of camera is however undesirable for the reasons noted above.

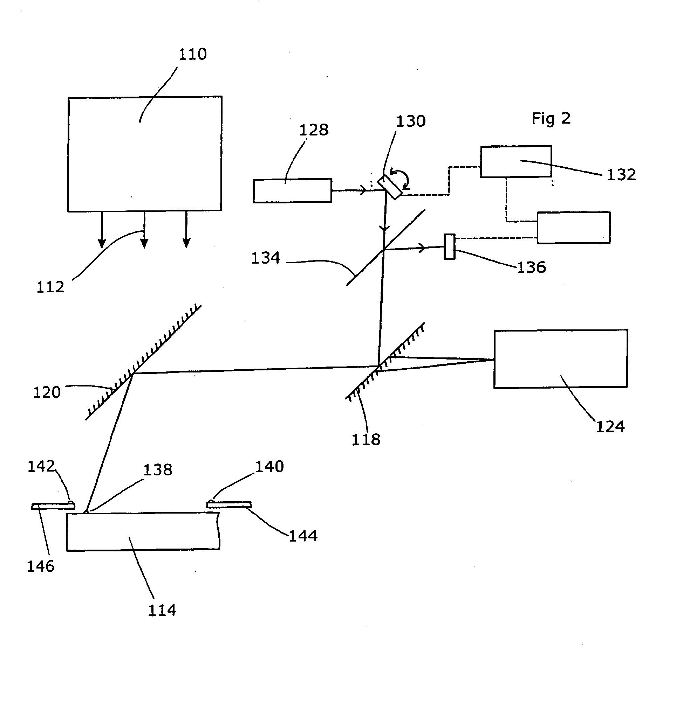

Figure 2 shows an embodiment of the present invention. As before, a radiation source 110 emits a beam of radiation 112 towards a treatment volume, and the shape of the radiation beam 112 is collimated by an MLC comprising an array of individual leaves 114. A laser light source 128 is also provided, directed towards a 2D MEMs (Micro-Electro-Mechanical systems) scanning mirror 130. This is controlled by a driver 132, which is programmed to cause the mirror 130 to oscillate so as to scan the laser light beam across each leaf 114 of the MLC, as described later. To direct the laser light to the leaves 114, mylar mirrors 118, 120 are provided as before.

A beam splitter 134 is placed in the path of the laser light, between the oscillating mirror 130 and the first mylar mirror. This directs at least some of the laser light returned from the leaves 114 to a photo sensor 136 capable of detecting the laser light.

On top of each leaf 114 is a retro-reflector 138 which reflects the laser light back in the direction from which it came, back towards the photo sensor 136. The time lapse between the laser light being reflected from the reference reflector and from the retro-reflector 138 is then used to determine the location of the retro-reflector 138, and thus the location of the leaf 114. The mirror then scans the laser beam along the length of the next leaf, repeating the process. In practice, the relevant position of the mirror is that which existed a few nanoseconds before receipt of the reflected light, to allow for travel time of up to about a metre in total. However, this small delay is unlikely to make a difference in practice.

A means for calibrating the motion of the MEMs within each scan cycle is also included. Additional reference reflectors 140, 142 are affixed just beyond the ends of each leaf 114 at the edges of the scan pattern, such as on the supports 144, 146 that surround the leaves 114. In this way, the position of the sinusoidal or other scanning maxima/minima can be measured during each leafpair traversal. This can, in turn, be used to calibrate for amplitude and frequency jitter during each scan cycle.

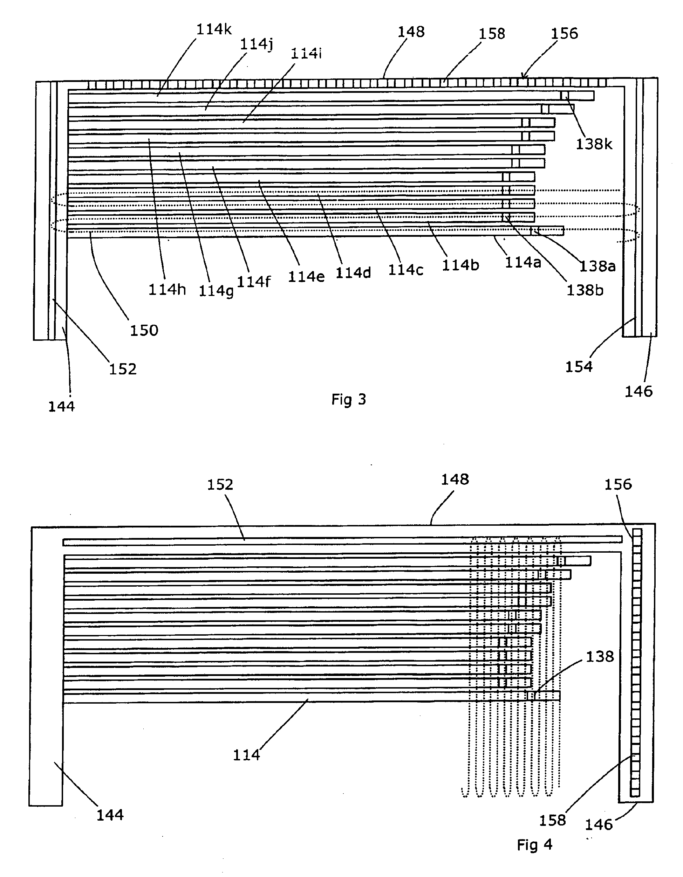

Figure 3 shows a suitable scan pattern. Viewed from along the radiation beam axis, the array of leaves 114a-114k sit alongside each other and are at varying positions. They are supported in a frame made up of the supports 144, 146 at either end of travel of the leaves 114 and a side member 148. Further leaves (in addition to those depicted in figure 3) sit alongside the illustrated leaves but are omitted for clarity; other elements of the frame are likewise omitted.

Each leaf 114a-114k has a corresponding reflector 138a-138k located at a known position along the leaf. Figure 3 illustrates a convenient location, in which the reflector is proximate to, but not at, the end of the leaf. Such a location reduces the risk of damage to the reflector during handling.

The oscillating mirror 130 scans the laser light along the serpentine path illustrated by dotted line 150. This takes the light beam along the length of each leaf 114 and will thus prompt a brief return signal as the light passes the respective reflector 138. The path has a maxima/minima that overlaps the supports 144, 146 at either end of travel of the leaves 114, at which points a continuous transverse reflector strip 152, 154 is provided. Thus, there is also a brief return signal at either end of the oscillation. This allows the oscillations to be calibrated, as the time delay between these signals should be constant and predictable, and also provides a frame around the return from the reflector 138 allowing it to be interpreted according to normal principles of pulse position modulation.

Alternatively, a raster pattern can be adopted.

The system also includes an in-built calibration for each scan cycle, to take into account any effects arising from possible non-linearities due to jitter and the sinusoidal nature of the scanning mirror's motion in the fast scanning axis (along the length of the MLC leaves). These effects are calibrated for by the use of one or more fixed calibration strips 156 upon which are mounted a series of reference reflectors 158 in a known pattern on the calibration strip. In this way, a comparison can be made with the theoretical sinusoidal motion of the MEMs mirror 130, and any deviations can be calibrated for. This also eliminates any drift that may arise from changes in temperature or wear in the scanning mirror mechanism over time. As illustrated in figure 3, the calibration strip is placed on the side member 148, but could be located elsewhere within the field of view of the detector 136. More than one such calibration strip could be provided.

Figure 4 illustrates an alternative scan pattern, also serpentine but transverse to the leaves 114 instead of along the leaves 114. Such a pattern would essentially scan through all the possible leaf positions and report which leaf was in that position, rather than scanning along each leaf and reporting the position of that leaf.

The following advantages can be gained from such a system;
- Low cost: none of the active components need to be radiation hard as they can be located away from the direct beam, and are sufficiently small that they can be easily shielded. The mirror device is based upon standard silicon processes and is geared towards mass production. Such a system could easily be 10% of the cost of current camera based systems.
- High reliability: The scanning mirror devices are fabricated on silicon using standard processes. There is little or no friction to cause wear, therefore the expected lifetime of the scanning mirror is likely to be in excess of the lifetime of the Linac. The remaining components have no moving parts and can expect to have similar lifetimes. Additionally, the system could be made in such a way that it would not be susceptible to radiation, meaning that (potentially) the components may never need replacement.
- Higher accuracy. The accuracy of the system is limited only by the jitter of the controlling electronics, which can be as low as 0.02%. This offers greater potential accuracy when compared with traditional camera based systems. The resolution of the system can easily exceed that of the current camera based system.
- Higher scan rate. The achievable scan rate can easily exceed the 30Hz currently available via a camera based system, allowing faster leaf movements to be tracked safely with improved control.
- Lower processing requirements. The algorithm used to determine leaf position is a simple one based on interpolation between the calibration points and the known scan pattern (such as sinusoidal), thus eliminating the need for complex image processing and fast processors to cope with the video data (most of which is discarded).
- Smaller size. The scanning mirror device itself is very small (typically 10mm²). The laser source and the photo sensor are also similarly small in size, thus reducing space requirements for the system.
- Reduced interference. The system could be made such that it only responds to one wavelength of light. Filtering this wavelength from entering or leaving the head would be a simple matter, requiring only the necessary optical filters. Additionally, due to the nature of the signals generated (effectively puise position modulation), the system's susceptibility to electromagnetic interference could be reduced. By using coded pulses of laser light, false pulses from electromagnetic or optical sources could be effectively eliminated.
- Redundancy. Due to the small size and low cost of the proposed system, an identical backup system could easily be employed to detect failures.

It will of course be understood that many variations may be made to the above-described embodiment without departing from the scope of the present invention.

## Claims

1. A multi-leaf collimator comprising;
an array of laterally spaced elongate leaves (114) each having a longitudinal edge on which is mounted a reflective element (138);
a scanning light source (128, 130) adapted to issue a beam of light in a scanning pattern, so that the beam moves over the longitudinal edges of a plurality of leaves (114) of the array; and
a detector (136) for light reflected from the reflective elements (138).

2. A multi-leaf collimator according to claim 1 in which the scanning light source comprises a source of light that illuminates a mirror (130), the mirror (130) being controllably adjustable so as to direct a reflected beam of light in a scan pattern.

3. A multi-leaf collimator according to claim 2 in which the mirror (130) is part of a micro-electromechanical device.

4. A multi-leaf collimator according to any one of claims 1 to 3 further comprising at least one mirror (140, 142) locatable in a radiation beam path to permit location of the scanning light source out of the beam path.

5. A multi-leaf collimator according to any one of claims 1 to 4 in which the scanning pattern illuminates a point that moves along the longitudinal edges of a plurality of leaves (114) of the array in succession.

6. A multi-leaf collimator according to any one of claims 1 to 5 in which the scanning light source comprises a laser.

7. A multi-leaf collimator according to any one of claims 1 to 6 in which the reflective element (138) is retro-reffective.

8. A multi-leaf collimator according to any one of claims 1 to 7 in which the leaves are mounted in a frame (144, 146), on which is mounted at least one reflective object (152, 154).

9. Apparatus comprising a multi-leaf collimator according to any one of claims 1 to 8, adapted to repeat the scanning pattern.

10. Radiotherapeutic apparatus comprising a multi-leaf collimator according to any of claims 1-8.

## Patentansprüche

1. Mehrblättrige Kollimator, aufweisend:
ein Feld von seitlich beabstandeten langgezogenen Blättern (114), die jeweils eine Längskante aufweisen, an der ein reflektierendes Element (138) angebracht ist;
eine Abtastlicfitquelte (128, 130), die dazu geeignet ist, einen Lichtstrahl in ein Abtastmuster auszustrahlen, so dass sich der Strahl über die Längskanten einer Vielzahl von Blättern (114) des Feldes bewegt; und
einen Detektor (136) für von den reflektierenden Elementen (138) reflektiertes Licht.

2. Mehrblättriger Kollimator nach Anspruch 1, wobei die Abtastlichtquelle eine Lichtquelle aufweist, die einen Spiegel (130) beleuchtet, wobei der Spiegel (130) durch Steuerung derart einstellbar ist, dass er einen reflektierten Lichtstrahl in ein Abtastmuster lenkt.

3. Mehrblättriger Kollimator nach Anspruch 2, wobei der Spiegel (130) Teil einer mikroelektromechanischen Vorrichtung ist.

4. Mehrblättriger Kollimator nach einem der Ansprüche 1 bis 3, ferner aufweisend mindestens einen Spiegel (140, 142), der in einem Strahlungsweg angeordnet werden kann, um die Anordnung der Abtastlichtquelle außerhalb des Strahlungswegs zu ermöglichen.

5. Mehrblättriger Kollimator nach einem der Ansprüche 1 bis 4, wobei das Abtastmuster einen Punkt beleuchtet, der sich nacheinander entlang der Längskanten einer Vielzahl von Blättern (114) des Feldes bewegt.

6. Mehrblättriger Kollimator nach einem der Ansprüche 1 bis 5, wobei die Abtastlichtquelle einen Laser aufweist.

7. Mehrblättriger Kollimator nach einem der Ansprüche 1 bis 6, wobei das reflektierende Element (138) retroreflektierend ist.

8. Mehrblättriger Kollimator nach einem der Ansprüche 1 bis 7, wobei die Blätter in einem Rahmen (144, 146) montiert sind, an dem mindestens ein reflektierendes Objekt (152, 154) montiert ist.

9. Gerät, aufweisend einen mehrblättrigen Kollimator nach einem der Ansprüche 1 bis 8, das dazu geeignet ist, das Abtastmuster zu wiederholen.

10. Strahlentherapeutisches Gerät, aufweisend einen mehrblättrigen Kollimator nach einem der Ansprüche 1 bis 8.

## Revendications

1. Collimateur multilames comprenant :
un agencement de lames allongées espacées latéralement (114) ayant chacune un bord longitudinal sur lequel est monté un élément réfléchissant (138);
une source de lumière de balayage (128, 130) adaptée pour émettre un faisceau de lumière selon un modèle de balayage, de sorte que le faisceau se déplace sur les bords longitudinaux d'une pluralité de lames (114) de l'agencement ; et
un détecteur (136) pour la lumière réfléchie à partir des éléments réfléchissants (138).

2. Collimateur multilames selon la revendication 1, dans lequel la source de lumière de balayage comprend une source de lumière qui éclaire un miroir (130), le miroir (130) étant ajustable de manière contrôlable de façon à diriger un faisceau réfléchi de lumière selon un modèle de balayage,

3. Collimateur multilames selon la revendication 2, dans lequel le miroir (130) fait partie d'un dispositif microélectromécanique,

4. Collimateur multilames selon l'une quelconque des revendications 1 à 3, comprenant en outre au moins un miroir (140, 142) qui peut être situé dans une trajectoire de faisceau de rayonnement pour permettre de situer la source de lumière de balayage en dehors de la trajectoire de faisceau.

5. Collimateur multilames selon l'une quelconque des revendications 1 à 4, dans lequel le modèle de balayage éclaire un point qui se déplace le long des bords longitudinaux d'une pluralité de lames (114) de l'agencement à la suite.

6. Collimateur multilames selon l'une quelconque des revendications 1 à 5, dans lequel la source de lumière de balayage comprend un laser.

7. Collimateur multilames selon l'une quelconque des revendications 1 à 6, dans lequel l'élément réfléchissant (138) est rétroréfléchissant.

8. Collimateur multilames selon l'une quelconque des revendications 1 à 7, dans lequel les lames sont montées dans un châssis (144, 146), sur lequel est monté au moins un objet réfléchissant (152, 154).

9. Appareil comprenant un collimateur multilames selon l'une quelconque des revendications 1 à 8, adapté pour répéter le modèle de balayage.

10. Appareil radiothérapeutique comprenant un collimateur multilames selon l'une quelconque des revendications 1 à 8.
